# EUROPEAN PATENT APPLICATION

(11) **EP 4 376 309 A1**
(43) Date of publication of application: **29.05.2024**
(21) Application number: 23206499.8
(22) Date of filing: 27.10.2023
(51) Int. Cl.: H04B 1/38, G16H 20/30

(54) **ADVANCED DATA TRANSMISSION SYSTEM BETWEEN A SUPERVISOR AND A PATIENT, PARTICULARLY FOR EDUCATIONAL AND THERAPEUTIC ASPECTS**

(30) Priority: 25.11.2022 IT 202200024351
(71) Applicant: Lombardo, Daniele, 95030 Sant'Agata li Battiati (CT) (IT); Lombardo, Marco, 95030 Sant'Agata li Battiati (CT) (IT)
(72) Inventor: Lombardo, Daniele, 95030 Sant'Agata li Battiati (CT) (IT); Lombardo, Marco, 95030 Sant'Agata li Battiati (CT) (IT)
(74) Representative: Bellomia, Paolo

(57) **Abstract**

Described is an advanced data transmission system comprising **a supervisor device (1)** associated with a supervisor user (S) and **a virtual meeting room** (2) associated with a patient (P) wherein the virtual meeting room (2) comprises a control device (30), associated with a therapist user (T) of the patient (P), at least one device for detecting data (10 i:1..n) produced by the patient (P), and a processing unit (100) associated with the supervisor device (1) configured to manage a receiving/transmitting and processing of the data exchanged between the supervisor (S) and the patient (P).

## Description

This invention relates to an advanced data transmission system, between a supervision unit and a patient, in particular for educational and therapeutic aspects.

In particular, the invention relates to an advanced data transmission system which is able to implement, manage, acquire data, measure, and facilitate the interaction and efficiency between a supervisor and a patient with reference to educational and therapeutic aspects.

There are prior art data transmission systems for interaction with a patient such as:
- the Ask Nao software for managing a humanoid robot produced by Aldebaran Robotics, developed in collaboration with the University of Birmingham Autism Centre, which stimulates the attention of children with autism with a series of games and educational exercises focused on the development of verbal and non-verbal communication;
- the Buddy Special Needs software, designed to help autistic children become more autonomous;
- the Robots4Autism software, which supports therapists, teachers and parents set up to operate with a robot, Milo (RoboKind), which is able to capture the attention of younger children;
- the Saracen project (Miur - Cnr Messina and Lecce) relative to a software to improve social and cognitive interactions of children with autism;
- the management software of a Kaspar robot (humanoid robot designed in 2005 by the English University of Hertfordshire and used as part of the Aurora project) which studies the impact of robots in treatments for autism.

Patent documents are also known which describe basic systems for data transmission with a patient.

The prior art basic data transmission systems suffer from a multiplicity of drawbacks linked mainly to a lack of computational resources dedicated to an interaction between patient and machine which simulates the interaction between the patient and a human supervisor.

In other words, these systems are inefficient in terms of the means for generating machine-patient interactions specifically in relation to educational and therapeutic aspects.

In particular, these prior art systems are inefficient in allowing a significant exchange of data of the conditions of the patient by means of a dedicated interface, in guaranteeing a capacity for voice recognition and interaction with the robots, in guaranteeing a capacity for interaction with the surrounding environment by the robots and in the management of several robots from a single software platform.

The aim of this invention is to overcome the drawbacks of the prior art.

The general aim of this invention is to provide an advanced data transmission system, in particular for educational and therapeutic aspects, which is efficient to operate, that is to say, optimised in terms of data exchange and response times with the patient.

A particular aim of the invention is to provide an advanced data transmission system, in particular for educational and therapeutic aspects, which allows an advanced processing of activities to be performed in enabling and rehabilitation processes of a patient.

These and other aims are achieved by an advanced data transmission system for generating interaction between a supervisor user and a patient, in particular for educational and therapeutic aspects, as described in the appended claims. According to the invention, the advanced data transmission system comprises a **supervisor device** associated with a supervisor user and a **virtual meeting room** associated with a patient wherein the virtual meeting room comprises a control device, associated with a therapist user of the patient, at least one device for detecting data produced by the patient, and a processing unit associated with the supervisor device configured to manage a receiving/transmitting and processing of the data exchanged between the supervisor and the patient.

The invention, as described, has the following technical effects:
- efficient operation of the receiving/transmitting, that is, optimised operation in terms of data exchange and response times between a supervisor and a patient;
- advanced processing of data of activities to be performed in enabling and rehabilitation processes of a patient.

The technical effects/advantages mentioned and the other technical effects/advantages of the invention are described in more detail below in the description of an embodiment illustrated by way of general example in the accompanying drawings.

Figure 1 is a block diagram of the system according to the invention.

Figure 2 is a block diagram of a detailed block of Figure 1.

The advanced data transmission system for generating interaction between a supervisor user and a patient, according to the invention, allows data to be acquired by the supervisor relating to a behaviour in the therapeutic/enabling field of a patient, whether this data is requested by a medical operator present, also known as a therapist, who acts for the patient, or it is requested by a robot, previously associated with the patient and usually controlled remotely by the supervisor.

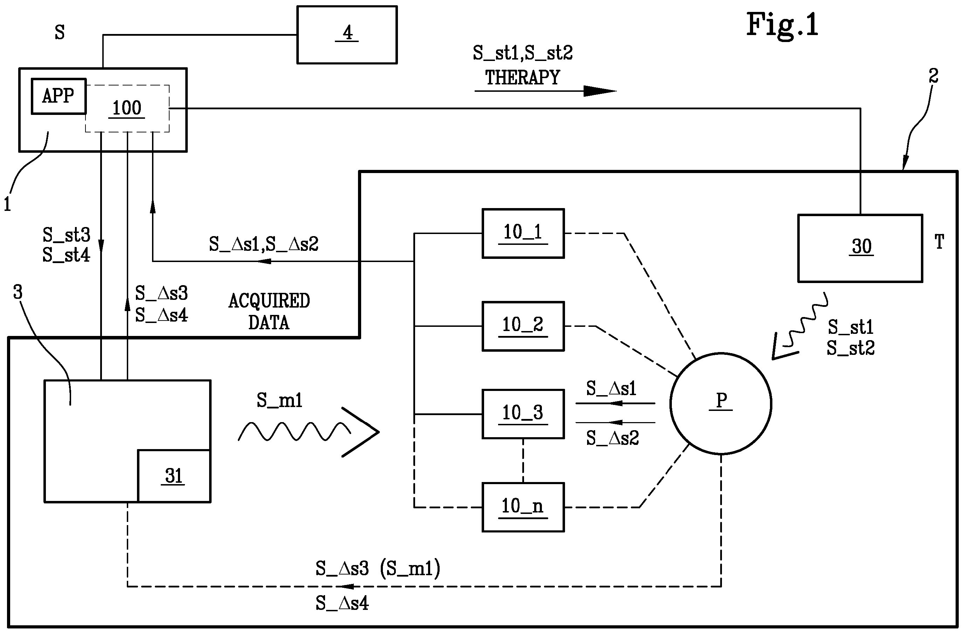

With reference in particular to Figure 1, the advanced data transmission system for generating interaction between a supervisor user S and a patient P comprises a **supervisor device 1** associated with the supervisor user S.

Preferably, the supervisor device 1 comprises an software application APP which comprises a specific user access by means of authentication.

Preferably, the supervisor device 1 comprises a tablet.

Preferably, the application APP is developed for Apple iPad devices and for iPadOS operating system.

The application APP is a specific interface for the supervisor, which performs the function of operator/educator, and is connected to a cloud memory unit 4 where all the information relating to the profiles of the patients P is stored in the log of the activities performed, in particular by means of a therapist or humanoid robot as described below, as well as the results obtained from these activities.

The supervisor device 1 is in data connection with a cloud memory unit 4 containing information on patients P and respective therapies.

The system according to the invention also comprises a **virtual meeting room 2** associated with the patient P for an interaction with the supervisor device 1. **According to a first embodiment of the invention,** the virtual meeting room 2 comprises a control device 30, associated with a therapist user T of the patient P, with data connection with the supervisor device 1.

Preferably, the control device 30 comprises headphones for receiving/transmitting information.

Alternatively or in addition, the control device 30 may comprise other devices designed to allow a communication with the supervisor device 1.

According to the invention, the control device 30 is configured to receive first signals representing the therapy stimuli S_st1 from the supervisor device 1, in particular from the application APP, for a stimulation of the patient P.

In this way, the therapy activity may be performed remotely using the supervisor device 1 which can conduct and process the therapy activities giving instructions and a support to the therapist T positioned alongside the patient, through the control device 30.

According to the invention, the virtual meeting room 2 also comprises at least one device for detecting data 10_i (i=1..n), produced by the patient P, wherein the data detection device 10_i is in data communication with the supervisor device 1. Preferably, the data detection device 10_i comprises one or more between a tablet PC, a smartphone, a PC, a smartwatch or the like.

In order to perform the two-way transmission of data between the supervisor device 1, the control device 30 and at least one data detection device 10_i, these devices set up a videoconference system wherein the patient P interacts with the supervisor device 1.

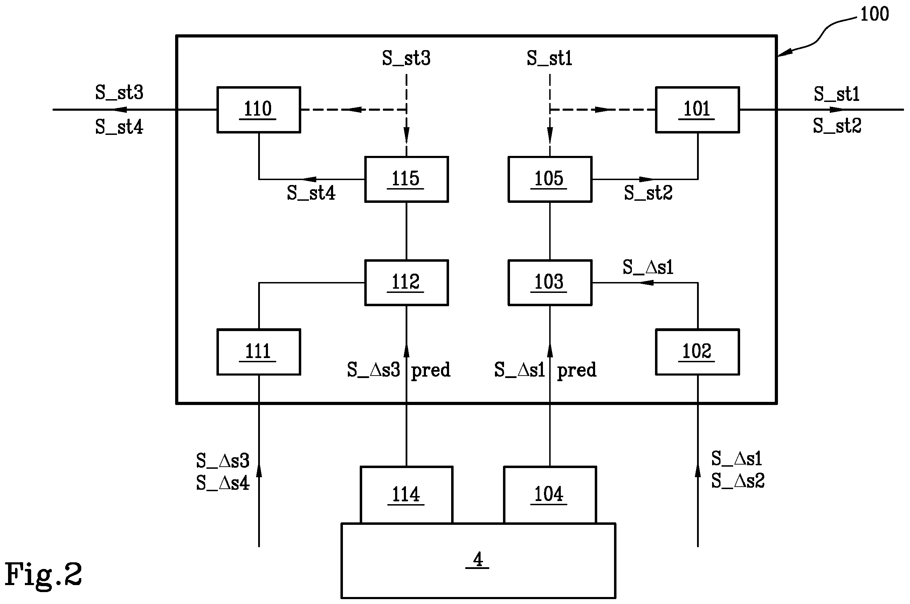

The supervisor device 1, in particular the software application APP, controls a processing unit 100, shown in general in Figure 1 and in detail in Figure 2, configured for receiving/transmitting and processing data exchanged between the supervisor device 1 and the devices included in the meeting room.

In general it should be noted that, in this context and in the following claims, the processing unit 100 is presented as divided into separate functional modules (memory modules or operating modules) for the sole purpose of describing the functionality in a clear and complete manner.

In reality, the processing unit may, in one case, consist of a single electronic device, suitably programmed to perform the functions described and the various modules can correspond to hardware units and/or software routines forming part of the programmed device.

Alternatively or in addition, the functions can be performed by a plurality of electronic devices on which the above-mentioned functional modules can be distributed.

The processing unit 100 may also have one or more processors for the execution of the instructions contained in the memory modules.

The above-mentioned functional modules may also be distributed on different computers, either locally or remotely, on the basis of the architecture of the network on which they are housed.

The processing unit 100, which is associated with the supervisor device 1 is configured to send first signals representing therapy stimuli S_st1; a transmission module 101, shown in Figure 2, performs this function.

In particular, the first signals representing therapy stimuli S_st1 are sent to the control device 30 for a corresponding communication to the patient P.

The patient P will react to the stimuli with signals representing changes in his/her state S_Δs1, in particular comprising signals representing movements of the face and/or vocal and/or aptic signals.

According to the invention, the at least one data detection device 10_i is configured for detecting the first signals representing status variations S_Δs1 emitted by said patient P and for transmitting the first signals representing status variations S_Δs1 to the supervisor device 1

The processing unit 100 of the supervisor device 1 is configured to receive the first signals representing predetermined status variations S_Δs1; a reception module 102, shown in Figure 2, performs this function.

The processing unit 100 is also configured for comparing the first signals representing status variations S_Δs1 with first predetermined signals representing possible status variations S_Δs1_pred; a comparison module 103, shown in Figure 2, performs this function.

Preferably, first predetermined signals representing possible status variations S_Δs1_pred are included in a memory module 104 associated with the cloud memory unit 4.

The processing unit 100 is also configured for varying the first signals representing therapy stimuli S_st1 sent on the basis of the comparison performed, in such a way as to generate second signals representing updated therapy stimuli S_st2; a processing module 105 performs this function.

In other words, on the basis of the reaction S_Δs1 of the patient P to the stimuli received S_st1 and the comparison with predetermined reactions, the processing unit 100 is configured for assessing the efficiency of the therapy in progress and adjusting a new phase of therapy signals comprising a new emission of stimuli S_st2 in order to improve the efficiency of the response of the patient P. Alternatively or in addition, the supervisor S can emit new stimuli S_st2 on the basis of the reaction S_Δs1 of the patient P.

The processing unit 100 is also configured to send the second signals representing updated therapy stimuli S_st2; the transmission module 101, shown in Figure 2, performs this function.

The patient P will react to the new stimuli with signals representing changes in his/her state S_Δs2, in particular comprising signals representing movements of the face and/or vocal and/or aptic signals.

According to the invention, the at least one data detection device 10_i is configured for detecting the second signals representing status variations S_Δs2 emitted by said patient P and transmitting the second signals representing status variations S_Δs2 to the supervisor device 1

The processing unit 100 of the supervisor device 1 is configured to receive the second signals representing predetermined status variations S_Δs2; the reception module 102, shown in Figure 2, performs this function.

**According to a second embodiment of the invention,** the supervisor device 1 can also directly stimulate the patient P by means of a remote operation using a robot remotely and therefore stimulate the execution of specific exercises as described above with reference to the control device 30, with consequent generation of signals representing movements of the face and/or voice and/or aptic signals by the patient P.

For this purpose, as schematically shown in Figure 1, the system according to the invention, in particular the meeting room 2, comprises a **humanoid robot 3** designed for an interaction with the patient P and the supervisor device 1.

According to this embodiment of the invention, the supervisor device 1, the control device 30, the at least one data detection device 10_i and the humanoid robot 3 form a video conference system for an interaction of the patient P with the supervisor device 1.

The data transmission system according to the invention is designed mainly for a supervisor/educator device that wants to use the robot to follow patients with neurodevelopmental deficit and dementia where the humanoid robot represents an engagement strategy which can be actuated to accelerate and make more efficient the enabling and rehabilitation processes of a patient.

In fact, the system according to the invention is a tool which the supervisor/educator uses for setting up the activities on the humanoid robot which will then be performed in therapy to be performed with an increasing efficiency. According to the invention, the humanoid robot 3 is designed to operate in various modes.

In a first mode, the so-called remote operation mode, the humanoid robot 3 is controlled totally by the supervisor device 1 by means of a plurality of signals representing therapy stimuli, for example S_st3, S_st4 as described below, received in succession and activators of all its functions.

In other words, in the remote operation mode the supervision device 1, by means of the application APP, totally controls the robot; in this mode, the supervisor has a panel for controlling the robot which provides for total operation.

This mode is usually used during the initial step of approaching the patient when a familiarisation activity is performed between robot and patient; the purpose of this is to make the robotic instrument known to the patient and also assess the relative level of understanding and interest by the patient.

In a second mode, the so-called autonomous mode, the humanoid robot 3 is pre-programmed for operation and partly controlled by the supervisor device 1 by means of a plurality of signals representing therapy stimuli, for example S_st3, S_st4 as described below, designed to select specific training content for the patient P.

In other words, the autonomous mode, after programming the robot, comprises engaging the robot with the patient autonomously without the intervention of the supervisor. The application APP allows a management and maintenance of the content/functions which the robot can perform; amongst this content, the supervisor can select the most suitable ones on the basis of the training requirements of the user for whom the engagement is performed.

Once the activity has been selected the operator launches the execution of a training session, the robot performs the activity according to a predetermined sequence and operates according to a question/answer exercise logic.

In a third mode, the so-called mixed mode, the humanoid robot 3 is partly pre-programmed for the operation, as in the autonomous mode, and partly controlled by the supervisor device 1, as in the remote operation mode.

In other words, the Mixed or Hybrid mode is the combination of the two described above, the robot performs an exercise/function, and during the performance of the latter, the supervisor, by means of the application APP, can remotely operate the robot modifying its behaviour/functions in such a way as to make it as interactive as possible with regard to the patient, especially when it is necessary to cover stimuli or responses not provided for in the programming of the exercise in progress.

According to the invention, with particular reference to Figure 1, the humanoid robot 3 is configured to receive third signals representing therapy stimuli S_st3 from the supervisor device 1, generating humanoid movements S_m1 for a stimulation of the patient P and detecting third signals representing status variations S_Δs3 emitted by the patient P, wherein the third signals are generated at least as a function of the humanoid movements S_m1.

In particular, the signals representing status variations S_Δs3 comprise signals representing movements of the face and/or voice and/or aptic signals emitted by the patient P.

The humanoid robot 3 according to the invention comprises a video camera 31 designed to detect the signals representing status variations.

The transmission system according to the invention comprises, as described and according to various modes, that the anthropomorphic robot 3 is controlled by the supervisor device 1, in particular by the associated processing unit 100.

In particular, as shown in Figure 2, the processing unit 100 is configured to send the third signals representing the therapy stimuli S_st3 to the humanoid robot 3; a transmission module 110, shown in Figure 2, performs this function.

The processing unit 100 is also configured for receiving from the humanoid robot 3 third signals representing status variations S_Δs3 determined as a function of the third signals representing the therapy stimuli S_st3 and for comparing the third signals representing status variations S_Δs3 with third predetermined signals representing possible status variations S_Δs3_pred; the functions are performed respectively by a reception module 111 and by a comparison module 112, shown in Figure 2.

Preferably, third predetermined signals representing possible status variations S Δs3_pred are included in a memory module 114 associated with the cloud memory unit 4.

The processing unit 100 is also configured for varying the third signals representing the therapy stimuli S_st3 sent on the basis of the comparison performed, in such a way as to generate fourth signals representing updated therapy stimuli S_st4; a processing module 115 performs this function.

In other words, based on the reaction S_Δs3 of the patient P to the stimuli received S_st3 and the comparison with predetermined status variations S_Δs3_pred, the processing unit 100 is configured for assessing the efficiency of the therapy in progress and adjusting a new phase of therapy signals comprising a new emission of stimuli S_st4 in order to improve the efficiency of the response of the patient P.

Alternatively, or additionally, the supervisor S can emit new stimuli S_st4 based on the reaction S_Δs3 of the patient P.

The processing unit 100 is also configured for sending to the humanoid robot 3 the fourth signals representing updated therapy stimuli S_st4 and receiving from the humanoid robot 3 fourth signals representing updated status variations S_Δs4 as a function of the fourth signals representing updated therapy stimuli S_st4; the functions are performed, respectively, by a transmission module 110 and by a reception module 111, shown in Figure 2.

The processing unit is also configured to record the signals representing updated status variations S_Δs3, S_Δs4 in the cloud memory unit 4 for further comparisons if necessary.

Generally speaking, in the system according to the invention, as described, the supervisor device performs a plurality of coordinated and coordinating functions. More specifically, the supervisor device 1 is configured for:
- preparing an activity comprising the therapy stimuli S_st1, S_st2, S_st3, S_st4 designed to be sent to the patient P.

In other words, starting from the patient records, the supervisor defines a programming for the intervention/functions and data recovery, where it specifies which behaviours to solicit and which observations to make with regard to the patient.

The supervisor device 1 is also configured for:
- starting a remote teleconferencing session enabling the control device 30, the at least one data detection device 10_i and/or the humanoid robot 3.

In other words, once the session has been created, an identifier of the meeting room is automatically generated where an operator or parent can also participate. The use of a video conferencing system allows the supervisor to observe the patient from various points of view, in which each point of view may be an endpoint connected to the meeting room generated; preferably, an endpoint may be a smartphone, a tablet, a PC, a smartwatch or the like;

The supervisor device 1 is also configured for:
- sending the signals representing the therapy stimuli S_st1, S_st2, S_st3, S_st4 to the control device 30, and/or to the humanoid robot 3.

In other words, the therapist who operates in situ with the patient can receive instructions using the headphones, that is to say, using the control device 30, from the supervisor for carrying out the therapy and therefore the stimulations to be performed.

The operator/supervisor may also control the robot, using the remote operation mode, and can therefore generate therapy stimuli and detect the patient's feedback by means of each data/endpoint detection device available.

The supervisor device 1 is also configured for:
- conducting and supervising the remote session using the at least one data detection device 10_i and/or the humanoid robot 3.

In other words, once the video conferencing session has started, the supervisor can observe the viewpoints (detection devices) linked to the video conferencing session and perform the data detection.

At the same time, it can operate with the robot by remote operation by means of the relative controls and observing the point of view of the robot through the integrated video cameras.

The supervisor operations may be performed with two supervisor devices 1 or with a single device. Using two devices makes it possible to always have available the remote operation panel of the robot and the feedback data receiving panel, which makes the supervisor's operations faster and more efficient and with more points of view always available.

The supervisor device 1 is also configured for:
- collecting and analysing the data received in the form of the signals representing status variations S_Δs1, S_Δs2, S_Δs3, S_Δs4.

In other words, the data acquired is stored in the user profile of each patient being managed by the operator/supervisor, in particular in the cloud memory unit 4.

An analysis panel can display the behaviour of the patient and the intervention times performed.

An advanced data transmission system has been described for generating interaction between a supervisor user and a patient, in particular for educational and therapeutic aspects.

The invention, as described, has the following technical effects:
- efficient operation of the receiving/transmitting, that is, optimised operation in terms of data exchange and response times between a supervisor and a patient;
- advanced processing of data of activities to be performed in enabling and rehabilitation processes of a patient.

## Claims

1. An advanced data transmission system for generating interaction between a supervisor user (S) and a patient (P) comprising:
- **a supervisor device (1)** associated with said supervisor user (S);
- **a virtual meeting room (2)** associated with said patient (P) for said interaction with said supervisor device (1), wherein said virtual meeting room (2) comprises: (P);
- a control device (30), associated with a therapist user (T) of said patient (P), in data connection with said supervisor device (1), wherein said control device (30) is configured to receive first signals representing therapy stimuli (S_st1) from said supervisor device (1) for stimulation of said patient (P);
- at least one device for detecting data (10_i; i=1..n), produced by said patient (P), in data communication with said supervisor device (1), wherein said at least one device for detecting data (10_i; i=1..n) is configured for
• detecting first signals representing status variations (S_µs1) emitted by said patient (P);
• transmitting said first signals representing status variations (S_µs1) to said supervisor device (1)
- a processing unit (100) associated with said supervisor device (1) and configured for
• sending (101) said first signals representing therapy stimuli (S_st1);
• receiving (102) said first signals representing status variations (S_µs1) determined as a function of said first signals representing therapy stimuli (S_st1);
• comparing (103) said first signals representing status variations (S_µs1) with first predetermined signals representing possible status modifications (104);
• based on said comparison, varying (105) said first signals representing therapy stimuli (S_st1) sent, in such a way as to generate second signals representing updated therapy stimuli (S_st2);
• sending (101) said second signals representing updated therapy stimuli (S_st2);
• receiving (102) second signals representing updated status variations (S_µs2) as a function of said second signals representing updated therapy stimuli (S_st2).

2. The system according to claim 1, wherein said supervisor device (1), said control device (30) and said at least one data detection device (10_i; i=1..n) form a videoconference system for an interaction of said patient (P) with said supervisor device (1).

3. The system according to claim 1 or 2, comprising:
- **a humanoid robot (3)** set up for an interaction with said patient (P) and said supervisor device (1), wherein said humanoid robot (3) is configured for
• receiving third signals representing therapy stimuli (S_st3)_from said supervisor device (1),
• generating humanoid movements (S_m1) for a stimulation of said patient (P);
• detecting third signals representing status variations (S_µs3) emitted by said patient (P), wherein said third signals are generated at least as a function of said humanoid movements (S_m1).

4. The system according to claim 3, wherein said processing unit (100) is also configured for:
• sending (110) said third signals representing therapy stimuli (S_st3) to said humanoid robot (3);
• receiving (111) from said humanoid robot (3) said third signals representing status variations (S_µs3) determined as a function of said third signals representing therapy stimuli (S_st3);
• comparing (112) said third signals representing status variations (S_µs3) with second predetermined signals representing possible status variations (114);
• varying (115) said third signals representing therapy stimuli (S_st3) sent on the basis of said comparison, in such a way as to generate fourth signals representing updated therapy stimuli (S_st4);
• sending (110) to said humanoid robot (3) said fourth signals representing updated therapy stimuli (S_st4);
• receiving (111) from said humanoid robot (3) fourth signals representing updated status variations (S_µs4) as a function of said fourth signals representing updated therapy stimuli (S_st4).
• recording said signals representing updated status variations (S_µs3,S_µs4).

5. The system according to claim 3 or 4, wherein said humanoid robot (3) comprises a video camera (31) designed to detect said signals representing status variations (S_µs3, S_µs4).

6. The system according to claim 3 or 4 or 5, wherein said humanoid robot (3) is set up to operate in one of the three modes:
- remote operation mode, wherein said humanoid robot (3) is fully controlled by said supervisor device (1) using a plurality of said signals representing therapy stimuli (S_st3,S_st4) received in succession and activating all its functions;
- autonomous mode, wherein said humanoid robot (3) is pre-programmed for operation and partly controlled by said supervisor device (1) using a plurality of said signals representing therapy stimuli (S_st3,S_st4) designed to select specific training content for said patient (P);
- mixed mode, wherein said humanoid robot (3) is partly pre-programmed for operation and partly controlled by said supervisor device (1) using a plurality of said signals representing therapy stimuli (S_st3,S_st4) designed to select specific training content for said patient (P).

7. The system according to any one of claims 3 to 6, wherein said supervisor device (1), said control device (30), said at least one data detection device (10_i; i=1..n) and said humanoid robot (3) form a videoconference system for an interaction of said patient (P) with said supervisor device (1).

8. The system according to any one of the preceding claims, wherein said control device (30) comprises headphones for transceiving information.

9. The system according to any one of the preceding claims, wherein said at least one data detection device (10_i; i=1..n) comprises one or more between a tablet PC, a smartphone, a PC, a smartwatch or the like.

10. The system according to any one of the preceding claims, wherein said signals representing status variations (S_µs1, S_µs2, S_µs3, S_µs4) comprise signals representing movements of the face and/or voice and/or haptic signals emitted by said patient (P).

11. The system according to any one of the preceding claims, wherein said supervisor device (1) is configured for
- preparing an activity comprising said therapy stimuli (S_st1,S_st2,S_st3,S_st4) designed to be sent to said patient (P);
- starting a remote teleconferencing session enabling said control device (30), said at least one data detection device (10_i; i=1..n) and/or said humanoid robot (3);
- sending said signals representing therapy stimuli (S_st1,S_st2,S_st3,S_st4) to said control device (30), and/or to said humanoid robot (3);
- conducting and supervising the remote session using said at least one data detection device (10_i; i=1..n) and/or said humanoid robot (3);
- collecting and analysing the data received in the form of said signals representing status variations (S_µs1, S_µs2, S_µs3, S_µs4).
